# EUROPÄISCHE PATENTSCHRIFT

(11) **EP 1 273 276 B1**
(45) Veröffentlichungstag und Bekanntmachung des Hinweises auf die Patenterteilung: **03.08.2005**
(21) Anmeldenummer: 02010894.0
(22) Anmeldetag: 16.05.2002
(51) Int. Cl.: A61F 2/44

(54) **Bandscheibenprothese**
Intervertebral disc prosthesis
Prothèse de disque intervertébral

(30) Priorität: 05.07.2001 DE 10132588
(43) Veröffentlichungstag der Anmeldung: 08.01.2003
(73) Patentinhaber: Fehling AG, 4153 Reinach BL 1 (CH)
(72) Erfinder: Fehling, Gerald, 63791 Karlstein (DE); Fehling, Guido, 63791 Karlstein (DE)
(74) Vertreter: Patentanwälte Westphal, Mussgnug & Partner

(56) Entgegenhaltungen:
- EP-A- 0 950 389
- DE-A- 10 130 825
- FR-A- 2 734 148
- FR-A- 2 775 587
- US-A- 5 716 416

## Beschreibung

Die Erfindung betrifft eine Bandscheibenprothese gemäß dem Oberbegriff des Anspruchs 1.

Die Bandscheiben dienen als druckelastische Abstützung zwischen den Wirbelkörpern der Wirbelsäule. Schäden der Bandscheiben, insbesondere durch Degeneration und Verschleiß können zu schweren Einschränkungen der Beweglichkeit und neurologischen Symptomen, insbesondere Schmerzen und Lähmungen führen. Sind solche Erkrankungen nicht mehr konservativ zu behandeln, so ist es bekannt, die Wirbelkörper miteinander zu verblocken. Dies hat allerdings zur Folge, dass die betroffenen Wirbel nicht mehr gegeneinander beweglich sind, so dass sich eine Versteifung der Wirbelsäule ergibt.

Als Alternative kann die defekte Bandscheibe durch eine gattungsgemäße Bandscheibenprothese ersetzt werden. Eine solche Bandscheibenprothese besteht aus einer oberen kranialen Scheibe und einer unteren kaudalen Scheibe, zwischen welcher Federmittel eingesetzt sind, die diese Scheiben druckelastisch gegeneinander abstützen. Die Bandscheibenprothese wird anstelle der entfernten Bandscheibe zwischen die Wirbel eingesetzt, wobei sie mit den Scheiben an den Wirbelkörpern des darüberliegenden bzw. des darunterliegenden Wirbels verankert werden. Bei einer aus der EP 0 706 354 B1 bekannten Bandscheibenprothese werden die Federmittel durch ein elastisches Kunststoffkissen gebildet. Bei einer aus der FR 2 734 148 A1 bekannten Bandscheibenprothese werden die Federmittel durch eine Feder aus Titan oder Stahl gebildet, deren Kennlinie ein exponentielles Ansteigen der Federkraft mit abnehmendem Wirbelabstand bewirkt. Diese bekannten Bandscheibenprothesen ermöglichen eine Axial- und Torsionsbeweglichkeit der Wirbel. Nachteilig ist, dass das Material der Federmittel Ermüdungserscheinungen aufweist, die zu einem Nachlassen der Funktionsfähigkeit der Bandscheibenprothese und bei metallischen Federn sogar zum Bruch führen können.

Der Erfindung liegt die Aufgabe zugrunde, eine Bandscheibenprothese zu schaffen, welche eine gute Beweglichkeit der Wirbel mit einer langen Funktionsfähigkeit vereinigt.

Diese Aufgabe wird erfindungsgemäß gelöst durch eine Bandscheibenprothese mit den Merkmalen des Anspruchs 1.

Vorteilhafte Ausführungen der Erfindung sind in den Unteransprüchen angegeben.

Der wesentliche Gedanke der Erfindung besteht darin, die Federmittel der Bandscheibenprothese aus einer Memory-Metalllegierung herzustellen, die bei Körpertemperatur superelastische Eigenschaften aufweist. Die Superelastizität, die auch als Pseudoelastizität bezeichnet wird, beruht auf einer spannungsinduzierten Umwandlung von Austenit in Martensit. Diese reversible Umwandlung der Kristallstruktur ermöglicht eine wesentlich größere elastische Verformung im Vergleich zu der Hookschen Elastizität herkömmlicher Materialien und insbesondere herkömmlicher Metalle, wie z. B. Stahl oder Titan. Da die superelastische Verformung auf einer Umwandlung der Kristallstruktur beruht und nicht wie bei der herkömmlichen Hookschen Elastizität auf einer Verformung der Kristallstruktur, führt die superelastische Verformung nicht zu einer Materialermüdung. Die elastischen Eigenschaften der Bandscheibenprothese bleiben daher auch bei langer Implantationsdauer und einer entsprechend hohen Zahl von Lastwechseln unverändert und ein Ermüdungsbruch der Federmittel ist nicht zu befürchten.

Die Verwendung von Memory-Metalllegierungen für die Herstellung der Federmittel erlaubt eine große Freiheit in der Gestaltung der Federmittel. Diese können in Bezug auf ihre elastischen Eigenschaften und ihre Federkraft fein abgestuft werden. Es ist eine optimale Anpassung der Federkennlinie sowohl bezüglich Axialbewegungen als auch bezüglich Torsionsbewegungen der Wirbel möglich.

In einer Ausführung können die Federmittel als Schraubendruckfedern ausgebildet sein. Eine solche Schraubendruckfeder ergibt eine besonders günstige Beweglichkeit der Wirbel in Bezug auf axiale Bewegungen, Kippbewegungen und Torsionsbewegungen.

Werden die Federmittel durch Tellerfedern gebildet, so läßt sich eine gute Axial- und Kippbeweglichkeit, gegebenenfalls mit höherer Steifigkeit realisieren, wobei keine oder nur eine geringe Torsionsbeweglichkeit vorhanden ist.

In einer weiteren Ausführung können die Federmittel durch eine Schraubenfeder gebildet sein, die in Form eines Torus zwischen den Scheiben liegt. Eine solche Schraubenfeder lässt eine axiale Beweglichkeit durch Zusammendrücken der Schraubenfeder senkrecht zu ihrer Achse zu. Ein Kippen der Windungen der Schraubenfeder gegen ihre Achse ermöglicht eine Torsionsbewegung. Schließlich können die Federmittel auch als eine oder mehrere Blattfedern ausgebildet sein. Mit solchen Blattfedern lässt sich insbesondere eine weiche axiale Abfederung erzielen. Torsionsbewegungen sind je nach Breite der Blattfedern mehr oder weniger möglich.

Die Bandscheibenprothese wird vorzugsweise für den Bereich der Wirbelsäule eingesetzt, der eine besonders hohe Beweglichkeit erfordert, d. h. insbesondere für den Bereich der Lendenwirbel und der unteren Brustwirbel. Die Ausbildung und Dimensionierung der Federmittel kann dabei nach dem Einsatz der Bandscheibenprothese gewählt werden, insbesondere nach der erforderlichen axialen Elastizität und der gewünschten Torsionsbeweglichkeit.

Vorzugsweise werden die Federmittel aus der Memory-Metalllegierung durch einen flexiblen Schutzmantel umhüllt, um ein Einwachsen von Gewebe zu verhindern, welches die Federeigenschaften der Federmittel beeinträchtigen könnte. Der Schutzmantel besteht vorzugsweise aus einem biokompatiblen dauerelastischen Kunststoff.

Im Folgenden wird die Erfindung anhand von in der Zeichnung dargestellten Ausführungsbeispielen näher erläutert. Es zeigen:
- Figur 1: schematisch eine erste Ausführung der Bandscheibenprothese im Axialschnitt,
- Figur 2: eine entsprechende Darstellung einer zweiten Ausführung,
- Figur 3: eine entsprechende Darstellung einer dritten Ausführung und
- Figur 4: eine entsprechende Darstellung einer vierten Ausführung.

Die Bandscheibenprothese weist eine obere kraniale Scheibe 10 und eine untere kaudale Scheibe 12 auf. Die Scheiben 10 und 12 sind aus einem starren biokompatiblen Material gefertigt, vorzugsweise aus Titan. Die Querschnittsform der Scheiben 10 und 12 entspricht im Wesentlichen der Form der natürlichen Bandscheibe bzw. des Wirbelkörpers. An der äußeren Oberfläche der Scheiben 10 und 12 sind Krallen 14 angeformt, die dazu dienen, beim Einsetzen der Bandscheibenprothese zwischen zwei Wirbel die Scheiben 10 und 12 in den anliegenden Flächen der Wirbelkörper zu verankern. Die Scheiben 10 und 12 sind axial beabstandet und werden gegeneinander durch druckelastische Federmittel abgestützt.

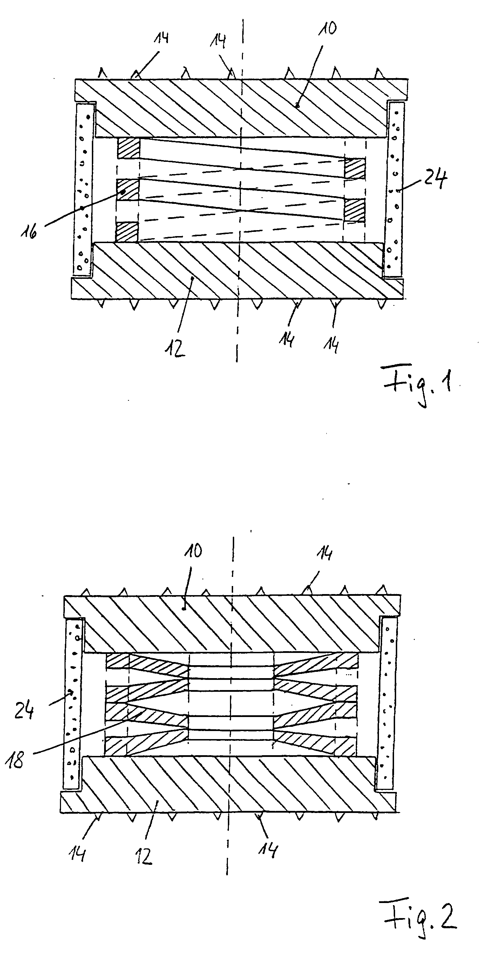

In dem Ausführungsbeispiel der Figur 1 sind diese Federmittel als Schraubendruckfeder 16 ausgebildet, in dem Ausführungsbeispiel der Figur 2 als ein Paket von Tellerfedern 18. Die Schraubendruckfeder 16 bzw. die Tellerfedern 18 sind achsmittig zwischen die Scheiben 10 und 12 eingesetzt. Die Scheiben 10 und 12 können auf diese Weise gegen den Druck der Schraubendruckfedern 16 bzw. der Tellerfedern 18 axial gegeneinander gedrückt und gegeneinander verkippt werden. Die Schraubendruckfeder 16 der Ausführung der Figur 1 läßt zudem eine gewisse gegenseitige Torsion der Scheiben 10 und 12 zu. Eine solche Torsion ist bei den Tellerfedern 18 nicht oder nur in sehr geringem Maße möglich.

In dem Ausführungsbeispiel der Figur 3 sind die Federmittel als Schraubenfeder 20 ausgebildet, die in Form eines Torus entlang dem Umfang der Scheiben 10 und 12 gelegt ist. Durch eine Verformung der Windungen der Schraubenfeder 20 können die Scheiben 10 und 12 axial gegeneinander gedrückt und gegeneinander verkippt werden. Eine Torsionsbewegung der Scheiben 10 und 12 ist möglich, indem die Windungen der Schraubenfeder 20 gegen deren Achse verkippt werden.

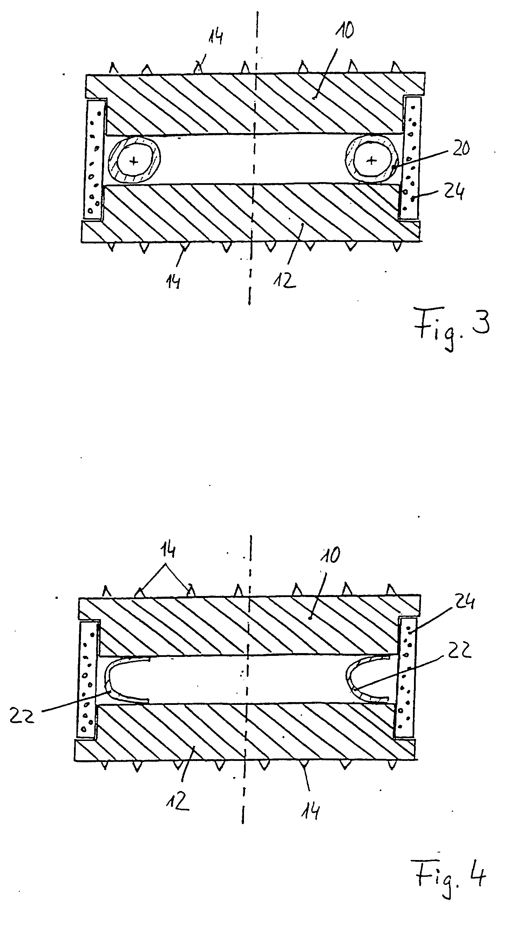

In der Ausführung der Figur 4 sind die Federmittel durch Blattfedern 22 gebildet. Diese Blattfedern 22 haben die Form von Streifen, deren eines Ende an der oberen Scheibe 10 und deren anderes Ende an der unteren Scheibe 12 angebracht ist. Die Blattfedern 22 sind U-förmig gebogen, wobei sie nach außen gewölbt sein können, wie dies in Figur 4 links gezeigt ist oder nach innen gewölbt sein können, wie dies in Figur 4 rechts gezeigt ist. Es sind wenigstens drei, vorzugsweise jedoch sechs oder mehr solcher Blattfedern 22 in gleichem Winkelabstand entlang dem Umfang der Scheiben 10 und 12 angeordnet. Die Blattfedern 22 ermöglichen eine besonders weiche axiale Abfederung, die insbesondere auch ein Verkippen der Scheiben 10 und 12 ermöglicht. Eine Torsionsbewegung ist nur möglich, wenn die Blattfedern 22 eine geringe Breite (in Umfangsrichtung der Scheiben 10 und 12) aufweisen.

Anstelle einzelner U-förmig gebogener Blattfedern 22, wie dies in Figur 4 dargestellt ist, kann auch eine einzelne Blattfeder vorgesehen sein, die achsmittig zwischen den Scheiben 10 und 12 angeordnet ist und über ihren Umfang in gleichem Winkelabstand verteilt radial nach außen gerichtete Arme aufweist, die alternierend an der oberen Scheiben 10 und an der unteren Scheibe 12 befestigt sind.

Die Federmittel 16, 18, 20, 22 sind aus einer Memory-Metalllegierung hergestellt, die im Bereich der Körpertemperatur (35°C bis 40°C) superelastische Eigenschaften aufweist. Eine solche Memory-Metalllegierung kann beispielsweise eine Nickel-Titan-Legierung sein.

Um ein Einwachsen von Gewebe in die Bandscheibenprothese zu verhindern, ist ein Schutzmantel 24 vorgesehen-. Der Schutzmantel 24 besteht aus einem biokompatiblen Kunststoff, der ausreichend weich und dauerelastisch ist, um die Beweglichkeit der Bandscheibenprothese nicht zu behindern.

In dem dargestellten Ausführungsbeispiel hat der Schutzmantel 24 die Form einer dünnwandigen zylindrischen Hülse, deren offene Enden jeweils auf die kraniale Scheibe 10 bzw. die kaudale Scheibe 12 aufgezogen und in geeigneter Weise abgedichtet und befestigt sind.

## Patentansprüche

1. Bandscheibenprothese mit einer kranialen Scheibe, mit einer zu der kranialen Scheibe im Wesentlichen parallelen und axial beabstandeten kaudalen Scheibe und mit zwischen diese Scheiben eingesetzten Federmitteln, welche diese Scheiben druckelastisch gegeneinander abstützen,
**dadurch gekennzeichnet, dass** die Federmittel (16, 18) aus einer Memory-Metalllegierung bestehen, die bei Körpertemperatur superelastische Eigenschaften aufweist.

2. Bandscheibenprothese nach Anspruch 1,
**dadurch gekennzeichnet, dass** die Federmittel (16, 18) aus einer superelastischen Nickel-Titan-Legierung bestehen.

3. Bandscheibenprothese nach Anspruch 1 oder 2,
**dadurch gekennzeichnet, dass** die Federmittel durch eine achsmittig zwischen den Scheiben (10, 12) angeordnete Schraubendruckfeder (16) gebildet sind.

4. Bandscheibenprothese nach Anspruch 1 oder 2,
**dadurch gekennzeichnet, dass** die Federmittel durch Tellerfedern (18) gebildet sind.

5. Bandscheibenprothese nach Anspruch 1 oder 2
**dadurch gekennzeichnet, dass** die Federmittel durch eine Schraubenfeder (20) gebildet sind, die in Form eines Torus zwischen den Scheiben (10, 12) angeordnet ist.

6. Bandscheibenprothese nach Anspruch 1 oder 2,
**dadurch gekennzeichnet, dass** die Federmittel durch eine oder mehrere gewölbte Blattfedern (22) gebildet sind.

7. Bandscheibenprothese nach einem der vorhergehenden Ansprüche,
**dadurch gekennzeichnet, dass** die Federmittel (16, 18, 20, 22) durch einen Schutzmantel (24) umschlossen sind.

8. Bandscheibenprothese nach Anspruch 7,
**dadurch gekennzeichnet, dass** der Schutzmantel (24) eine zylindrische Hülse aus einem weichen dauerelastischen biokompatiblen Kunststoff ist.

## Claims

1. An intervertebral disc prosthesis having a cranial disk, a caudal disc that is substantially parallel to the cranial disk and axially spaced from the cranial disk and spring means inserted between these discs, which support these discs elastically upon compression axially against each other,
**characterised in that** the spring means (16, 18) are made from a memory metal alloy, which has super-elastic properties at body temperature.

2. An intervertebral disc prosthesis according to Claim 1,
**characterised in that** the spring means (16, 18) are made from a super-elastic nickel-titanium alloy.

3. An intervertebral disc prosthesis according to Claim 1 or 2,
**characterised in that** the spring means are formed by a helical compression spring (16) which is disposed truly axially between the discs (10, 12).

4. An intervertebral disc prosthesis according to Claim 1 or 2,
**characterised in that** the spring means are formed by disk springs (18).

5. An intervertebral disc prosthesis according to Claim 1 or 2,
**characterised in that** the spring means are formed by a helical spring (20), which is disposed in the form of a torus between the discs (10, 12).

6. An intervertebral disc prosthesis according to Claim 1 or 2,
**characterised in that** the spring means are formed by one or more curved leaf springs (22).

7. An intervertebral disc prosthesis according to one of the preceding Claims,
**characterised in that** the spring means (16, 18, 20, 22) are enclosed by a protective coating (24).

8. An intervertebral disc prosthesis according to Claim 7,
**characterised in that** the protective coating (24) is a cylindrical sleeve made from a soft, permanently elastic, biocompatible plastic material.

## Revendications

1. Prothèse de disque intervertébral comprenant un disque crânien, un disque caudal pour l'essentiel parallèle et écarté axialement du disque crânien, et des moyens de ressort insérés entre ces disques et qui sollicitent ces disques l'un vis-à-vis de l'autre par pression élastique,
**caractérisée en ce que**
les moyens élastiques (16, 18) sont fabriqués à partir d'un alliage métallique à mémoire qui, à la température du corps, présente des propriétés superélastiques.

2. Prothèse de disque intervertébral selon la revendication 1,
**caractérisée en ce que**
les moyens élastiques (16, 18) sont fabriqués à partir d'un alliage nickel-titane superélastique.

3. Prothèse de disque intervertébral selon la revendication 1 ou 2,
**caractérisée en ce que**
les moyens élastiques sont un ressort de pression hélicoïdal (16) disposé au centre de l'axe entre les disques (10, 12).

4. Prothèse de disque intervertébral selon la revendication 1 ou 2,
**caractérisée en ce que**
les moyens élastiques sont des ressorts à disques (18).

5. Prothèse de disque intervertébral selon la revendication 1 ou 2,
**caractérisée en ce que**
les moyens élastiques sont un ressort hélicoïdal (20) disposé en forme d'un tore entre les disques (10, 12).

6. Prothèse de disque intervertébral selon la revendication 1 ou 2,
**caractérisée en ce que**
les moyens élastiques sont un ou plusieurs ressorts à lames (22) bombés.

7. Prothèse de disque intervertébral selon l'une quelconque des revendications précédentes,
**caractérisée en ce que**
les moyens élastiques (16, 18, 20, 22) sont entourés par une enveloppe de protection (24).

8. Prothèse de disque intervertébral selon la revendication 7,
**caractérisée en ce que**
l'enveloppe de protection (24) est un manchon cylindrique en matière plastique douce biocompatible à élasticité permanente.
